(19) Europäisches Patentamt
European Patent Office
Office européen des brevets

(11) **EP 4 282 953 A1**

(12) **EUROPEAN PATENT APPLICATION**
published in accordance with Art. 153(4) EPC

(43) Date of publication:
**29.11.2023 Bulletin 2023/48**

(21) Application number: **21921254.5**

(22) Date of filing: **07.12.2021**

(51) International Patent Classification (IPC):
***C12N 5/077*** *(2010.01)*

(52) Cooperative Patent Classification (CPC):
**C12N 5/06**

(86) International application number:
**PCT/JP2021/045015**

(87) International publication number:
**WO 2022/158152 (28.07.2022 Gazette 2022/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.01.2021 JP 2021006928**

(71) Applicant: **Regenesis Science Co., Ltd.**
**Fukuoka-shi, Fukuoka, 810-0044 (JP)**

(72) Inventors:
• **YANAGA Hiroko**
**Fukuoka-shi, Fukuoka 810-0001 (JP)**
• **YANAGA Katsu**
**Fukuoka-shi, Fukuoka 810-0001 (JP)**

(74) Representative: **Moser Götze & Partner**
**Patentanwälte mbB**
**Paul-Klinger-Strasse 9**
**45127 Essen (DE)**

(54) **METHOD FOR PRODUCING MATURE CHONDROCYTES AND MATURE CHONDROCYTE-CONTAINING COMPOSITION**

(57) [Problem] Provided is a method for producing a mature chondrocyte appropriate for a transplantation.

[Solution] A method for producing a mature chondrocyte includes a cartilage culturing step of culturing cells from cartilage using a first culture medium to obtain a mature chondrocyte. The first culture medium is a medium containing hydrocortisone and FGF-2.

Fig. 1

EP 4 282 953 A1

**Description**

**TECHNICAL FIELD**

The present invention relates to methods for producing mature chondrocyte and secreted tropic factors derived from the cell.

**BACKGROUND ART**

In the field of reconstructive surgeries, many reconstruction operations with bone and cartilage transplantation had been performed. For donors sites, a skull, a rib, a costal cartilage, an ilium bone, and the like have been used. However, a possibility that collecting a large amount of cartilages causes functional and morphological problems on the donor site cannot inevitable. In a case of requiring multiple times of reconstructive surgeries, many bone and cartilage collection parts are often used from cadavers. Additionally, there is a limitation in the collection amount.

Therefore, the inventors including Vacanti invented a tissue engineering method in which a cartilage is regenerated from a limited number of chondrocytes obtained by enzymatically treating a cartilage tissue of an animal. This method is an invention [1, 2] in which chondrocytes are seeded on a scaffold with a mesh structure formed of an artificially synthesized degradable polymer, and the cells adhere to and proliferate on the scaffold to regenerate the cartilage tissue. However, since the cartilage tissue was not regenerated with the cartilages uniformly in the scaffold, a clinically applicable result has not been obtained yet. Further, it was found that the synthetic polymer in the scaffold induced an inflammation when absorbed in vivo, thus causing the regenerated cartilage to be absorbed [3-4]. As reported by the inventors including Vacanti later, an artificial cartilage structure containing a large amount of cells usually needs a high oxygen environment for the growth and the proliferation. Accordingly, when the cartilage is not formed up to the center of the structure, a perichondrium is not formed [5]. Accordingly, the supply of blood and nutrients to them was considerably restricted. It was found that this caused the cell death and inevitable necrosis of the artificial structure, and eventually the shape and the function were lost.

Non-patent Document 1: Vacanti CA, Langer R, Schloo B, Vacanti JP. Synthetic polymers seeded with chondrocytes provide a template for new cartilage formation. Plast Reconstr Surg. 1991; 88:753-759

Non-patent Document 2: Cao Y, Vacanti JP, Paige KT, Upton J, Vacanti CA. Transplantation of Chondrocytes Utilizing a Polymer-Cell Construct to Produce Tissue-Engineered Cartilage in the Shape of a Human Ear. Plast Reconstr Surg. 1997; 100: 297-302

Non-patent Document 3: Santavirta S, Konttinen YT, Saito T, et al. Immune response to polyglycolic acid implants. J Bone Joint Surg Br. 1990; 72:597-600.

Non-patent Document 4: Neidel J, Zeidler U. Independent effects of interleukin 1 on proteoglycan synthesis and proteoglycan breakdown of bovine articular cartilage in vitro. Agents Actions. 1993; 39:82-90.

Non-patent Document 5: Vacanti CA. The history of tissue engineering. J Cell Mol Med 2006; 10: 569-576.

Non-patent Document 6: Yanaga H., Imai K., Koga M., Yanaga K. Cell-engineered human elastic chondrocytes regenerate natural scaffold in vitro and neocartilage with neo-perichondrium in the human body post-transplantation. Tissue Eng. Part A. 2012; 18:2020-2029

Non-patent Document 7: Yanaga H., Imai K., Fujimoto T., Yanaga K. Generating ears from cultured autologous auricular chondrocytes by using two-stage implantation in treatment of microtia. Plast Reconstr Surg. 2009; 124:817-825

**DISCLOSURE OF THE INVENTION**

**PROBLEMS TO BE SOLVED BY THE INVENTION**

Up to the present, the clinical application of the transplantation of cultured chondrocytes has been difficult because the sufficient cell mass for ensuring a tissue size necessary for the transplantation is not obtained. It is an object of the present invention to provide a method for producing a enough numbers of mature chondrocytes appropriate for a transplantation and factors from the cell which support chondrocyte survive in vivo after the transplantation.

**SOLUTIONS TO THE PROBLEMS**

A first invention described in this description relates to a method for producing a mature chondrocyte. The mature chondrocyte is applicable to, for example, a cartilage regeneration operation as described later.

The method for producing a mature chondrocyte includes a cartilage culturing step of culturing chondrocytes using a first culture medium to obtain a mature type of chondrocyte.

The first culture medium is a medium containing hydrocortisone and FGF-2.

An example of the cartilage is an auricular cartilage. The cartilage may be a shredded cartilage (micro cartilage).

The chondrocyte culturing step includes, for example, a step of culturing the chondrocytes under an environment of 2% or more and 15% or less of carbonic acid gas and 1% or more and 10% or less of oxygen gas against air. In the chondrocye culturing step, different media may be used between a primary culture and a subculture. For example, for the primary culture, a medium containing an autoserum and FGF-2 may be used. For example, when the mature chondrocyte is re-cultured for a certain period after a cryopreservation, a medium containing an autoserum and FBS, and hydrocortisone and FGF-2 may be used.

Another invention described in this description relates to a method for producing factors from cultured mature chondrocytes. The method includes: a chondrocyte culturing step from cartilage using a first culture medium to obtain a mature chondrocyte; and obtaining step of factors from mature chondrocyte. The first culture medium is a medium containing hydrocortisone and FGF-2.

An example of the harvesting cartilage is an auricular cartilage. The cartilage may be a shredded cartilage.

The chondrocyte culturing step includes a step of culturing cells from the cartilage under an environment of 2% or more and 15% or less of carbonic acid gas and 1% or more and 10% or less of oxygen gas against air. In the chondrocyte culturing step, different media may be used between a primary culture and a subculture. For example, for the primary culture, a medium containing an autoserum and FGF-2 may be used. For example, when chondrocyte is cultured for a certain period after a cryopreservation, a medium containing FBS, hydrocortisone, and FGF-2 may be used.

The conditioned medium contains interleukin 8 (IL-8), a growth-related oncogene (GRO), a monocyte chemotactic protein-1 (MCP-1), and Vascular Endothelial Growth Factor (VEGF).

The factors containing mature chondrocyte is used for a transplantation for a cartilage regeneration.

The factors from mature chondrocyte is a composition used for a pinnaplasty, an external ear canal construction, a rhinoplasty, a mandibular construction, a hard tissue depression construction operation of cheekbones, a hard tissue depression construction operation of skull, a hard tissue depression construction operation of trachea, funnel chest, or the like.

Another invention described in this description relates to a method for producing a revascularization promoter.

The revascularization promoters contains factors from mature chondrocyte , which contains a vascular inducers such as VEGF, GRO and a VEGFR2 positive cell.

The method includes a step of producing the factors secreted from mature chondrocyte by the method for producing factors from mature chondrocyte described above.

## ADVANTAGEOUS EFFECTS OF THE INVENTION

The invention can provide a method for producing a mature chondrocyte appropriate for a transplantation. For example, by the transplantation simultaneously adding the cultured chondrocyte and the conditioned medium (for example, supernatant liquid of the mature chondrocyte) at the transplantation, the regeneration of blood vessel around cartilage is increased to improve an engraftment rate of the chondrocyte transplantation, thus increasing the size of the transplanted cartilage and allowing longterm survival. Accordingly, since the transplanted regenerated cartilage is sufficiently supplied with blood and nutrients, cell death and necrosis are reduced, and then the shape and the function are maintained. Since the cartilage is supplied with nutrients, a large amount of chondrocytes are engrafted, and absorption of the cartilage is reduced, thus allowing obtaining a larger size of regenerated cartilage that cannot be obtained so far.

By simultaneously adding the cultured chondrocyte and the supernatant liquid produced by the cell at the transplantation, the cartilage formation is accelerated, and the regenerated cartilage is significantly increased, thus allowing the success of the transplantation. The transplantation of the chondrocyte together with the supernatant liquid (condition medium) causes neovascularization around the cartilage, thereby achieving the high transplantation rate. Then, by confirming what factors are present in the cultured cell, several cytokines/chemokines causing the blood vessel induction were specifically found at high concentrations. By adding the mature chondrocyte and a supernatant liquid (CM: conditioned medium) especially containing GRO, IL8, MCP-1, and VEGF among the specific cytokines/chemokines produced by the mature chondrocyte and accelerating the blood vessel formation to the chondrocyte, the absorption after the transplantation can be reduced, and the engraftment rate can be improved.

## BRIEF DESCRIPTION OF THE DRAWINGS

Fig. 1 is a photograph substituted for a drawing illustrating a phase contrast micrograph of a chondrocyte in Example 1.

Fig. 2 is a graph substituted for a drawing illustrating an analysis result of cytokine and chemokine contained in a conditioned medium (Conditioned Medium) obtained in a culture of a cultured mature chondrocyte.

Fig. 3 is a photograph substituted for a drawing illustrating a regenerated cartilage collected from an abdomen one year after a transplantation.

Fig. 4 includes photographs substituted for drawings illustrating results of a histopathological analysis.

Fig. 5 includes photographs substituted for drawings illustrating a change of VEGFR2 of a cultured chondrocyte in a subculture.

Fig. 6 is a graph substituted for a drawing illustrating the number of allowable subcultures of a chondrocyte and PDL.

Fig. 7 is a photograph substituted for a drawing illustrating a cultured mature chondrocyte (P = 13) of a 20-year-old female.

Fig. 8 is a photograph substituted for a drawing illustrating a cultured mature chondrocyte (P = 13) of a 63-year-old female.

Fig. 9 is a photograph substituted for a drawing illustrating a cultured mature chondrocyte (P = 14) of a 37-year-old female.

Fig. 10 is a photograph substituted for a drawing illustrating a cultured mature chondrocyte (P = 14) of a 29-year-old female.

## DESCRIPTION OF PREFERRED EMBODIMENTS

The following describes embodiments of the present invention using the drawings. The present invention is not limited to the embodiments described below and includes ones appropriately changed in an obvious range by those skilled in the art from the following embodiments.

A first invention described in this description relates to a method for producing a mature chondrocyte. The mature chondrocyte is applicable to, for example, a cartilage regeneration operation as described later.

The method for producing a mature chondrocyte includes a chodrocyte culturing step of culturing cells from cartilage using a first culture medium to obtain a mature chondrocyte.

Then, the first culture medium is a medium containing hydrocortisone and FGF-2. The medium may contain any or both of an autoserum and FBS.

An example of the cartilage is an auricular cartilage. The cartilage may be a shredded cartilage (micro cartilage). One obtained by shredding the cartilage, performing a cleaning, and then, removing blood components may be used.

The chondrocyte culturing step includes, for example, a step of culturing the cells from cartilage under an environment of 2% or more and 15% or less of carbonic acid gas and 1% or more and 10% or less of oxygen gas against air. In the chondrocyte culturing step, different media may be used between a primary culture and a subculture. For example, for the primary culture, a medium containing an autoserum and FGF-2 may be used. For example, when the the mature chondrocyte is re-cultured after a certain period after a cryopreservation, a medium containing an autoserum and FBS, and hydrocortisone and FGF-2 may be used.

Another invention described in this description relates to a method for producing a composition containing mature chondrocyte and the conditioned medium. The composition is used for a transplantation for a cartilage regeneration. The composition is used for a pinnaplasty, an external ear canal construction, a rhinoplasty, a mandibular construction, a hard tissue depression construction operation of cheekbones, a hard tissue depression construction operation of skull, a hard tissue depression construction operation of trachea, funnel chest, or the like.

Chondrocyte Culturing Step

The chondrocyte culturing step is a step of culturing cells from the cartilage using the first culture medium to obtain a mature chondrocyte. In the chondrocyte culturing step, a collected cells from cartilages may be directly cultured (after cleaning). The chondrocyte culturing step may be a step of culturing from a cut micro cartilage, or may be a step of culturing cells from shredded and filtered micro cartilage. The following description is based on the step of culturing cells from the shredded and filtered micro cartilage.

The first culture medium is a medium containing hydrocortisone and FGF-2. This medium may contain any or both of an autoserum and FBS. The first culture medium is preferably a medium containing an autoserum, hydrocortisone, and FGF-2 or a medium containing FBS, hydrocortisone, and FGF-2.

For the medium, it is only necessary to add a required component to a basic medium as necessary. Examples of the basic medium include $\alpha$-MEM medium, Eagle basic medium, and DMEM. As a reagent, it is only necessary to add publicly known one used for the medium as necessary. Examples of the reagent include fetal bovine serum (FBS), hydrocortisone (HC), FGF2, insulin-like growth factor (IGF), insulin, platelet derived growth factor (PDGF), adenocorticotropic hormone (ACTH), leukemia inhibitor factor (LIF), TGF$\beta$, BMP, steroid, chondroitin sulfate, soybean trypsin inhibitor, ascorbic acid, hyaluronic acid, proline, dexamethasone, insulin, transferrin, and selenious acid. When ascorbic acid or the like is added to the medium, one having a salt form, such as diphosphate, may be used. Each of them may be added to the medium so as to be 0.1 ng/mL or more and 20 $\mu$g/mL or less (or 0.2 ng/mL or more and 10 $\mu$g/mL or less). It is only necessary to add them with an appropriate adjustment depending on a degree of purification and the required amount. The autoserum may be added instead of FBS. A serum-free medium may be used. In any case, HC (hydrocortisone) and FGF-2 are preferably added to the medium.

An exemplary culture medium is one in which 1% to 10% of fetal bovine serum (FBS), 20 ng/ml or more and 100 ng/ml or less of hydrocortisone (Hydrocortisone), and from 5 ng/ml to 20 ng/ml (or 50 ng/ml) of FGF2 (Fibroblast Growth Factor 2) are added to $\alpha$-MEM medium. 1% to 10% of autoserum may be added together with or instead of 1% to 10% of fetal bovine serum (FBS). These amounts may be appropriately adjusted. For example, FBS and the autoserum may be contained in the medium by 0.1% to 20%.

The cells from micro cartilage may be cultured and proliferated under an ordinary culture condition. The cell mass is allowed from about 10% to a state of 100% confluent, and the culture in a high density and layered state to maturing of chondrocytes, for example, exceeding 100% confluent is also allowed. A transition to a hypoxic state may be made immediately after the transplantation or after leaving to stand for a while. The hypoxic culture may be performed, for example, by using a hypoxia incubator of a type of mixing a commercially available nitrogen gas or the like to reduce an oxygen partial pressure, or by blowing a nitrogen gas or the like into an appropriate space to reduce the oxygen partial pressure.

The chondrocyes culturing step includes, for example, a step of culturing the cells form cartilage under an environment of 2% or more and 15% or less of carbonic acid gas and 1% or more and 10% or less of oxygen gas. In the chondrocyte culturing step, different media may be used between a primary culture and a subculture. For example, for the primary culture, a medium containing an autoserum and FGF-2 may be used. For example, when the mature chondrocyte is re-cultured after a certain period after a cryopreservation, a medium containing FBS, hydrocortisone, and FGF-2 may be used.

Composition Containing Mature Chondrocyte and Conditioned Medium Obtaining Step

The composition containing mature chondrocyte and conditioned medium obtaining step is a step of obtaining a composition containing mature chondrocyte and conditioned medium obtained through the mature chondrocyte culturing step.

A preferred example of the mature chondrocyte is a cell that expresses each of interleukin 8 (IL-8), growth related gene (GRO), monocyte chemotactic protein-1 (MCP-1), and VEGF, which are chemokines/cytokines, by four times or more of IL-6. The cell may express any or two or more of IL-8, GRO, and MCP-1 by ten times or more of IL-6, may express by 20 times or more of IL-6, or may express by 20 times or more of IL-6. Thus, since the cytokine/chemokine that is high in revascularization promotion ability and low in inflammation is contained, the composition containing mature chondrocyte provides a high revascularization promotion ability without causing an inflammation in the transplantation, the composition containing mature chondrocyte is appropriate for the transplantation. The composition containing mature chondrocyte preferably contain a cartilage especially collected from a patient to which the transplantation is performed.

A preferred example of the mature chondrocyte is one that expresses any or two or more of interleukin 8 (IL-8), growth related gene (GRO), monocyte chemotactic protein-1 (MCP-1), and VEGF by ten times or more of TNF-$\alpha$, preferably expresses 50 times or more of TNF-$\alpha$, preferably expresses 100 times or more of TNF-$\alpha$, and preferably expresses 500 times or more of TNF-$\alpha$.

A preferred example of the mature chondrocyte is one that expresses any or two or more of interleukin 8 (IL-8), growth related gene (GRO), monocyte chemotactic protein-1 (MCP-1), and VEGF by ten times or more of IL-1-$\beta$, preferably expresses 50 times or more of IL-1-$\beta$, preferably expresses 100 times or more of IL-1-$\beta$, and preferably expresses 500 times or more of IL-1-$\beta$.

A preferred example of the mature chondrocyte is one that expresses any or two or more of interleukin 8 (IL-8), growth related gene (GRO), monocyte chemotactic protein-1 (MCP-1), and VEGF by ten times or more of INF-$\gamma$, preferably expresses 50 times or more of INF-$\gamma$, preferably expresses 100 times or more of INF-$\gamma$, and preferably expresses 500 times or more of INF-$\gamma$.

It is only necessary that the composition containing mature chondrocyte contains the mature chondrocyte and the conditioned medium (or culture supernatant) by appropriate amounts depending on the purpose. It is only necessary that the composition is housed in a required container similarly to an ordinary composition, thus allowing the use as necessary. The amount of the mature chondrocyte may be, for example, from $1 \times 10^4$ cells to $1 \times 10^{10}$ cells per use, or may be from $1 \times 10^5$ cells to $1 \times 10^8$ cells per use.

An agent containing the culture supernatant as an active ingredient is publicly known as disclosed in, for example, JP-A-2013-18756, Japanese Patent No. 5139294, and Japanese Patent No. 5526320. Accordingly, the composition containing the conditioned medium can be produced by using a publicly known method.

Examples of the culture supernatant of the mature chondrocyte include a processed product obtained by removing water content by lyophilization from a culture supernatant as a supernatant component obtained through a solid-liquid separation of a culture supernatant by centrifugation, a processed product obtained by a vacuum concentration of a culture supernatant using an evaporator or the like, a processed product obtained by concentrating a culture supernatant using an ultrafiltration membrane or the like, or a processed product obtained by a solid-liquid separation of a culture supernatant using a filter, or a stock solution of a culture supernatant before the above-described processes. Alternatively, an aseptic culture supernatant may be obtained, for example, by centrifuging (for example, 1,000×g, 10 minutes) a supernatant obtained by culturing the mature chondrocyte of the present invention, subsequently, performing ammonium sulfate fractionation (for example, 65% saturated ammonium sulfate), suspending the precipitate with an appropriate buffer solution and then performing a dialysis process, and performing filtering with a syringe filter (for example, 0.2 μm). The collected culture supernatant can be directly used, or can be cryopreserved and defrosted to be used when it is used. A pharmaceutically allowable carrier may be added to the culture supernatant, and the culture supernatant may be dispensed into a sterile container by an easily handled liquid volume. Further, as a countermeasure to a risk of an infectious agent, the culture supernatant may be processed with a virus clearance filter or by ultraviolet irradiation. The composition containing mature chondrocyte preferably contain the culture supernatant by 1 mL or more and 1, 000 mL or less in a unit of one administration, and further preferably 30 mL or more and 300 mL or less.

By the transplantation simultaneously adding the cultured chondrocyte and the conditioned medium (for example, supernatant liquid of the mature chondrocyte) at the transplantation, the regeneration of blood vessel around cartilage is increased to improve an engraftment rate of the chondrocyte transplantation, thus allowing significantly increasing the regenerated cartilage leading to the success of the transplantation. This composition is used for, for example, a pinnaplasty, a rhinoplasty, a mandibular construction, a hard tissue depression construction operation of cheekbones, a hard tissue depression construction operation of skull, and a hard tissue depression construction operation of funnel chest.

Another invention described in this description relates to a revascularization promoter. The revascularization promoter contains, for example, the composition containing mature chondrocyte obtained by any of the above-described methods for producing the composition containing mature chondrocyte. That is, the revascularization promoter is similar to the above-described composition containing mature chondrocyte. The revascularization promoter may be, for example, an injection preparation. It is only necessary that the composition containing mature chondrocyte is included in a syringe, mixed with a tissue of a patient as necessary, and transplanted to a required site of the patient. The revascularization promoter contains a composition containing mature chondrocyte that contains a vascular inducer and a VEGFR2 positive cell. Then, the method includes a step of producing a composition containing mature chondrocyte by the above-described method for producing the composition containing mature chondrocyte.

[Example 1]

An auricular cartilage of 1 square centimeter was collected from remains of an ear of a patient. The fraction was finely minced and rinsed with a phosphate buffered saline (Ca$^-$) supplemented with an antibiotic. Next, they were processed with 0.3% of collagenase (Worthington Biochemical, Freehold, NJ)/PBS, and rotated at 37°C for four to six hours by a stirrer, and then, chondrocyte was isolated. In a primary culture, the chondrocyte was seeded with a cell density of $1 \times 10^3$ cells/cm$^2$, and a DMEM medium to which, 10% of autologous serum and FGF-2 (5 to 10 ng/ml, FIBRAST (registered trademark), Kaken Pharmaceuticals, Tokyo) were added was used for the primary culture. In a subculture, the chondrocyte was seeded by $1 \times 10^3$ cells/cm$^2$ per 175 cm$^2$ culture flask. Next, the subcultured cell was seeded, thus obtaining a transplantation material containing the chondrocyte and CM as an end product ($1 \times 10'$ cells/1 cc CM). As the medium, one in which 5% of FBS (Fetal Bovine Serum), 40 ng/ml of Hydrocortisone, 10 ng/ml of Fibroblast Growth Factor 2 were added to High glucose-DME medium was used. As the culture condition, the culture was performed under 5% to 10% of carbonic acid gas against air. On the 12-th day of the primary culture, the cultured cells of 2 to $3 \times 10^6$ was collected. A part of the cells was frozen, and the other cells were seeded with a density of 4 to $5 \times 10^4$/cm$^2$ and cultured for 14 days. 15 days later, cleaning with PBS (-) was performed three times, and the culture on the medium in which FBS was removed from the above-described medium components was performed for two days. This medium was used for the analysis as a conditioned medium (Conditioned Medium).

Medium Composition

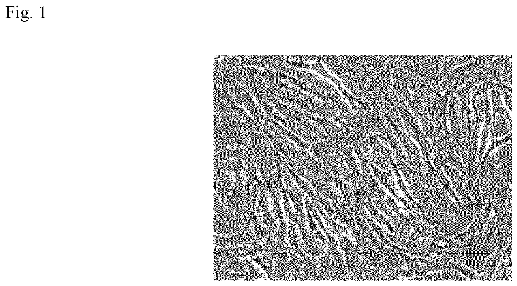

As the medium, one in which 5% of fetal bovine serum (FBS: Fetal Bovine Serum), 40 ng/ml of hydrocortisone (Hydrocortisone), 10 ng/ml of FGF2 (Fibroblast Growth Factor 2) were added to High glucose-DME medium was used. As the culture condition, the culture was performed under 10% of carbonic acid gas and 5% of oxygen gas. Fig. 1 is a photograph substituted for a drawing illustrating a phase contrast micrograph of the chondrocyte in Example 1.

[Example 2]

A cytokine/chemokine analysis of the conditioned medium (Conditioned Medium) of the cultured mature chondrocyte was performed by an antibody-immobilized magnetic beads method (Antibody-Immobilized Magnetic Beads method). A supernatant obtained by centrifuging a sample of the conditioned medium (Conditioned Medium) with 13, 000 G at 4°C for five minutes was uses for the measurement. Concentrations of 40 target proteins in the conditioned medium (Conditioned Medium) were measured using Luminex (registered trademark) system. A preprocessed sample was used by 25 $\mu$L per well, and the measurement was performed three times. One standard solution was added based on the manual, seven 5-fold dilution series were prepared, and the measurement was performed three times. The 40 items of the target proteins were EGF, FGF-2, eotaxin, TGF-$\alpha$, G-CSF, Flt-3L, GM-CSF, fractalkine, IFN$\alpha$2, IFN$\gamma$, GRO, IL-10, MCP-3, IL-12P40, MDC, IL-12P70, PDGF-AA, IL-13, PDGF-AB/BB, IL-15, sCD40L, IL-17A, IL-1RA, IL-1$\alpha$, IL-9, IL-1$\beta$, IL-2, IL-3, IL-4, IL-5, IL-6, IL-7, IL-8, IP-10, MCP-1, MIP-1$\alpha$, MIP-1$\beta$, RANTES, TNF$\alpha$, TNF$\beta$, and VEGF.

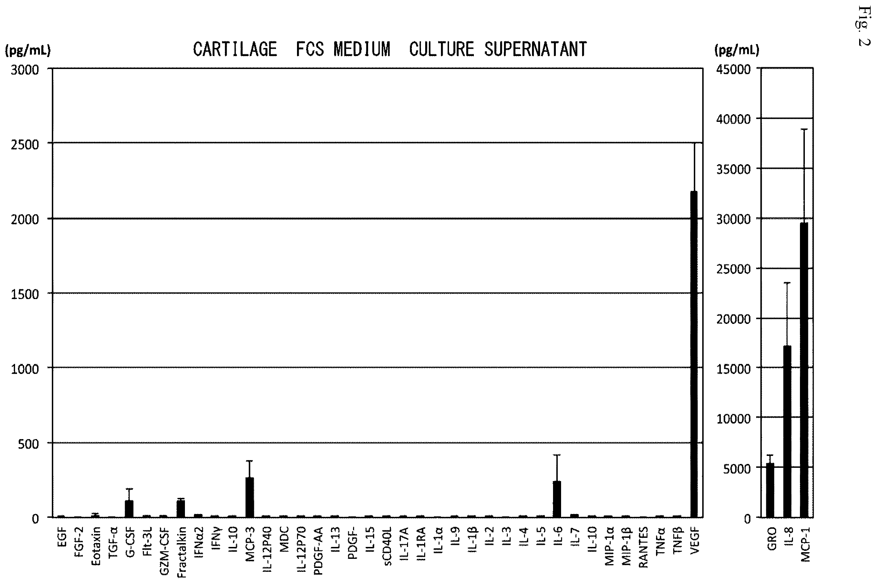

A cytokine/chemokine analysis of the conditioned medium (Conditioned Medium) of the cultured mature chondrocyte was performed by Antibody-Immobilized Magnetic Beads method. Fig. 2 illustrates the result. Fig. 2 is a graph substituted for a drawing illustrating the analysis result of cytokine and chemokine contained in the conditioned medium (Conditioned Medium) obtained in the culture of the cultured mature chondrocyte. As illustrated in Fig. 2, it was proved that a large amount of (ng/ml) IL-8, GRO, and MCP-1 that were cytokines having high revascularization ability was produced. Meanwhile, since almost no TNF-$\alpha$, which causes an inflammation, and IL-1-$\beta$ and INF-$\gamma$, which are highly inflammation inducible, were produced in the cell, the safety was demonstrated.

[Example 3]

Chondrocyte Transplantation to Human (Cartilage Formation for Auricular Reconstruction)

[Case 1]: 22-year-old male A reconstruction treatment using a cultured chondrocyte was performed because of bilateral microtia (missing external ears) and low formed cheekbones. A cartilage tissue of 15 mm $\times$ 15 mm was collected from the remaining right auricular cartilage, sterilized, and seeded and cultured. As the medium, one in which an autologous serum and 5% of FBS, hydrocortisone, FGF2 were added to High glucose-DME medium was used. The cultured cell of 4.3 $\times$ 10$^6$ cells was collected on the 11-th day of the primary culture, and the cultured cell of 2.2 $\times$ 10$^7$ cells was collected on the 13-th day of the primary culture. The cultured cells were cryopreserved once. Before the transplantation, the defrosted cells were cultured in 30 flasks having a bottom area of 150 cm$^2$ for 40 days. A composition containing the cultured mature chondrocyte and the conditioned medium (Conditioned Medium) was prepared by 230 cc (1 $\times$ 10$^7$ cells/1 cc CM) in total quantity. The composition containing the cultured mature chondrocyte and the conditioned medium (Conditioned Medium) was transplanted by injection to bilateral lower abdomen, and both auricles and cheekbones were reconstructed using a regenerated cartilage collected one year after the transplantation.

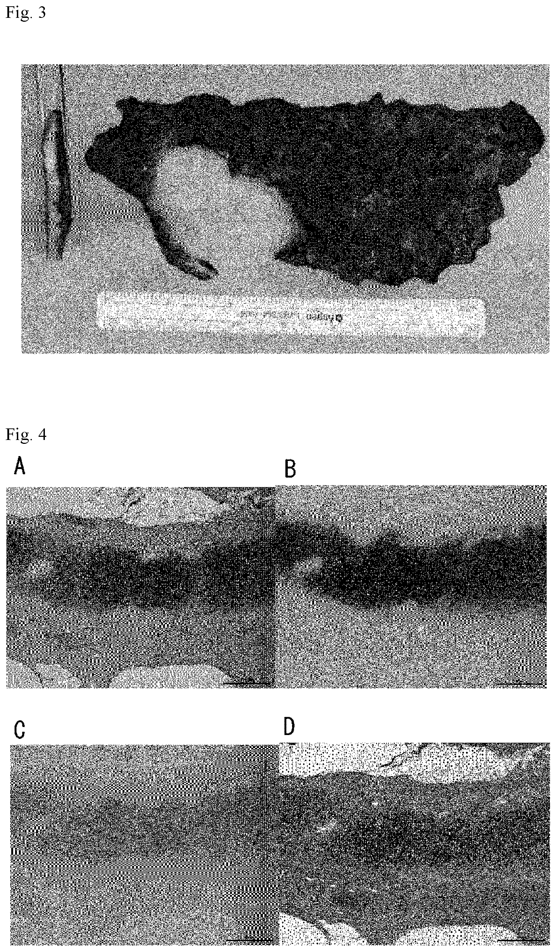

Fig. 3 is a photograph substituted for a drawing illustrating the regenerated cartilage collected from the abdomen one year after the transplantation. In Fig. 3, the size of the regenerated cartilage was 80 $\times$ 200 $\times$ 8 mm. A cut surface of the cartilage was a white cartilage tissue in the entire layer.

Next, a histopathological analysis was performed to show the regeneration of the cartilage. Fig. 4 illustrates the result. Fig. 4 includes photographs substituted for drawings illustrating the results of the histopathological analysis. Fig. 4A illustrates the result of HE staining. Fig. 4B illustrates the result of toluidine blue staining. Fig. 4C illustrates the result of alcian blue staining. Fig. 4D illustrates the result of Elastica van Gieson staining. As illustrated in Fig. 4A, a perichondrium is formed around the cartilage, and many vascular lumens are seen in the perichondrium. This indicates that nutrients are supplied from the perichondrium to the cartilage. From Fig. 4B to Fig. 4D, since the cartilages are stained by respective pigments that specifically stain the cartilages, it is seen that the cartilage is formed. As illustrated in Fig. 4, it is seen that a mature regenerated cartilage and a regenerated perichondrium are formed after the transplantation, and traits and character of an elastic cartilage tissue derived from the auricle are maintained.

[Example 4]

Chondrocyte Transplantation to Human: Funnel Chest Chest Depression Reconstruction

For an 18-year-old male, remaining of chest depression after the funnel chest surgery (from around midline xiphoid process to hypochondrium) was confirmed. A cartilage tissue of 10 mm $\times$ 7 mm was collected from the left auricle, sterilized, and seeded and cultured. As the medium, one in which an autologous serum, Hydrocortisone, and Fibroblast Growth Factor 2 were added to High glucose-DME medium was used. The cultured cell of 4.2 $\times$ 10$^6$ cells was collected on the 21-th day of the primary culture, and the cultured cell of 3.3 $\times$ 10$^6$ cells was collected on the 23-th day of the primary culture. The cultured cells were cryopreserved once. Before the transplantation, the defrosted cells were cultured in 15 flasks having a bottom area of 150 cm$^2$ for 40 days. The total quantity of the transplantation of the cultured mature chondrocyte and the condition medium was 84 mL ($1 \times 10^7$ cells/1 cc CM). For two years after the transplantation, the cartilage was not absorbed, and the appearance form of the chest was excellent.

[Example 5]

Chondrocyte Transplantation to Human: Nose Deformity, Depressed Deformation of Skull

20-year-old female, A reconstruction treatment was performed to forehead and nose with a cultured chondrocyte because of saddle nose, short nose, and depressed deformation of forehead. A cartilage tissue of 10 mm $\times$ 15 mm was collected from the left auricle, sterilized, and seeded and cultured. As the medium, one in which an autologous serum, Hydrocortisone, and Fibroblast Growth Factor 2 were added to High glucose-DME medium was used. The cultured cell of $1.1 \times 10^7$ cells was collected on the 18-th day of the primary culture. The cultured cells were cryopreserved once. Before the transplantation, the defrosted cells were cultured in seven flasks having a bottom area of 150 cm$^2$ for 40 days. The total quantity of the transplantation of the cultured mature chondrocyte and the conditioned medium was 27.3 mL ($1 \times 10^7$ cells/1 cc CM). For three years after the transplantation, the cartilage was not absorbed, and the appearance form was excellent.

[Example 6]

Expression of VEGFR2 of Cultured Cells in Subculture

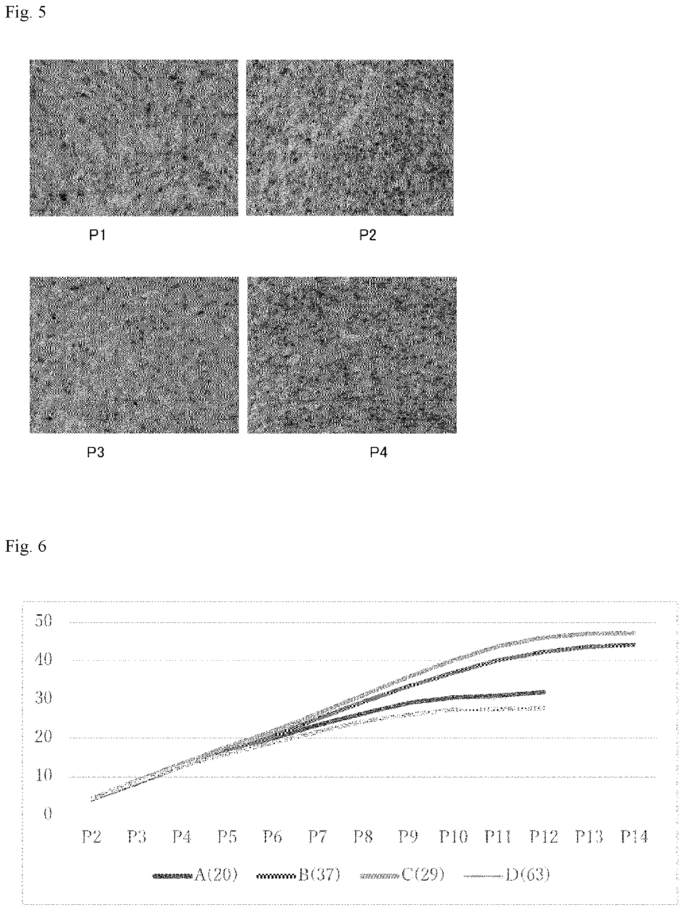

VEGFR2 is expressed only in a vascular endothelial cell and its precursor cell. Therefore, when VEGFR2 is expressed on the cultured cells , it is seen that when the cells are transplanted in patient's body , new blood vessels appears around formed cartilages. Those used for the transplantation are cells of P2 to P4. For VEGFR2, the cell that expresses VEGFR2 was present in all of P1 to P4. It was found that, in the culture system of chondrocyte, an endothelium-based cell was contained in all of P1 to P4. Accordingly, it can be expected that, by the transplantation of this cell system, the vascular endothelial cell appears together with the cartilage tissue, and consequently, a network of blood vessels is established around the cartilage tissue, thereby maintaining the cartilage tissue after the transplantation. Fig. 5 includes photographs substituted for drawings illustrating the expression of VEGFR2 of the cultured cells in the subculture.

[Example 7]

Next, to examine the number of allowable subcultures of the cultured chondrocyte, the number of allowable subcultures of the chondrocyte and PDL were measured. There is a characteristic that a tumorized cell does not stop proliferation but continues to proliferate. To confirm the safety of the cultured chondrocyte produced this time, aging of the chondrocyte was inspected. It is an inspection of whether the proliferation stops or not by culturing the cell and continuing the subculture.

Auricular cartilages of four persons were cultured for three months, and PDL (CPD) until the proliferation stops was measured. (It means how many times the division occurred.) Fig. 6 illustrates the result. Fig. 6 is a graph substituted for a drawing illustrating the number of allowable subcultures of the chondrocyte and PDL. As illustrated in Fig. 6, it was confirmed that the proliferation stopped due to Aging.

P14 = total 84 day culture: vertical axis CDL(= PDL). A (20-year-old), B (37-year-old), C (29-year-old), D (63-year-old)

Since cells exhibit positive staining by aging-related β-galactosidase (SA-β-gal) by senescence, activity staining by a senescence-associate enzyme Senescence-associate galactosidase staining was performed to the auricular cultured chondrocytes at the subculture of P13, P14 for the four persons to examine the cellular senescence, and consequently, as a result of the Senescence-associate galactosidase activity staining performed at each phase, since the enzyme activity appeared before the proliferation stop, it was confirmed that the proliferation was stopped by aging. For the Senescence-associate galactosidase activity staining, SA X-Gal staining Cellular Senescence Kit: OZBIOSCIENCES (Catalog Number: GXS0003) was used.

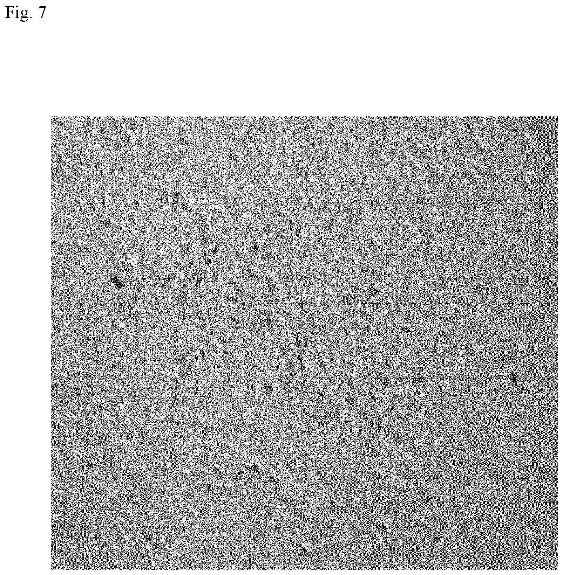

Fig. 7 is a photograph substituted for a drawing illustrating a cultured mature chondrocyte (P = 13) of a 20-year-old female.

A. 20-year-old female chondrocyte (proliferation stop at P-13)

$$\text{PDL} = 0.8952 \ (\text{P-12: } 2.5 \times 10^5 \Rightarrow 4.65 \times 10^5/\text{dish})$$

Total PDL: 27.6676 **SA X-Gal: 92% positive**

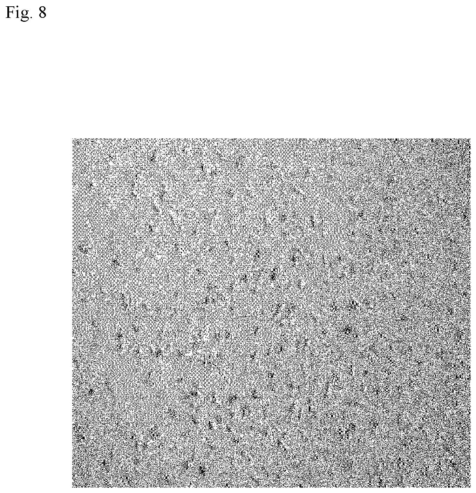

Fig. 8 is a photograph substituted for a drawing illustrating a cultured mature chondrocyte (P = 13) of a 63-year-old female.

D. 63-year-old female chondrocyte (proliferation stop at P-13)

$$PDL = 0.2388 \; (P\text{-}12: 2.5 \times 10^5 \Rightarrow 2.95 \times 10^5/\text{dish})$$

Total PDL: 27.6676 **SA X-Gal: 95% positive**

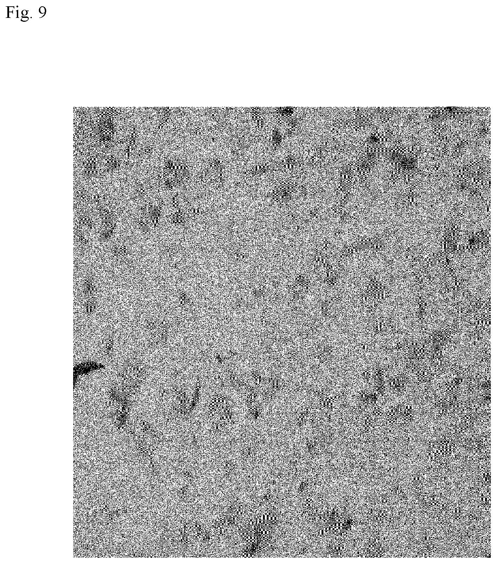

Fig. 9 is a photograph substituted for a drawing illustrating a cultured mature chondrocyte (P = 14) of a 37-year-old female.

B. 37-year-old female chondrocyte

$$PDL = 0.5109 \; (P\text{-}14: 1 \times 10^5 \Rightarrow 1.425 \times 10^5/\text{dish})$$

Total PDL: 44.0962 **SA X-Gal: 100% positive**

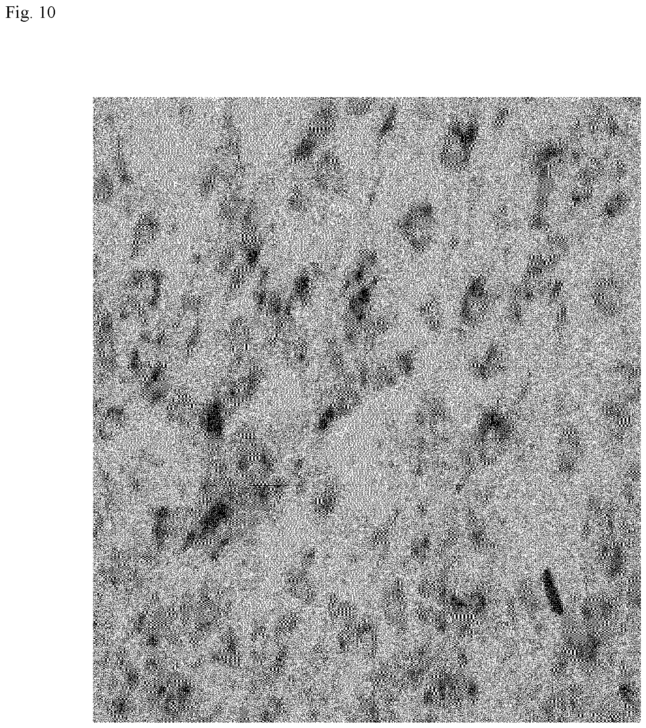

Fig. 10 is a photograph substituted for a drawing illustrating a cultured mature chondrocyte (P = 14) of a 29-year-old female.

C. 29-year-old female chondrocyte

$$PDL = 0.2013 \; (P\text{-}14: 1 \times 10^5 \Rightarrow 1.15 \times 10^5/\text{dish})$$

Total PDL: 47.1421 **SAX-Gal: 100% positive**

Examination

For the formation of the regenerated cartilage and then suppressing its absorption, formation of the perichondrium is necessary. A transplanted cartilage structure containing a large amount of cells usually requires a high oxygen environment for the growth and proliferation. Therefore, the cartilage is not formed up to the center of the structure when the perichondrium is not formed [5]. Accordingly, the supply of blood and nutrients to them were considerably restricted. It was found that this caused the cell death and inevitable necrosis of the transplantaed structure, and then, the shape and the function were lost [6-8]. Meanwhile, since cultured mature chondrocyte secretes cytokine/chemokine that has high revascularization promotion ability and low inflammation, the cultured mature chondrocyte provides a high revascularization promotion ability without causing an inflammation after the transplantation, and therefore, since a regenerated perichondrium is formed around the regenerated cartilage, the cultured mature chondrocyte is appropriate for the regenerating the cartilage after the transplantation. The conditioned medium of the cartilage contains VEGF by seven times of IL6, GRO by 20 times of IL6, IL8 by 80 times of IL6, and MCP-1 by 100 times of II,6. Since almost no TNF-$\alpha$, and IL-1-$\beta$ and INF-$\gamma$, which are highly inflammation inducible, were produced in the cell, the safety against immunological attacks was demonstrated. Since the proliferation stop by aging was ascertained in the Aging test, it was confirmed that the cell was not tumorized, and the safety was demonstrated.

The angiogenesis ability of IL-8 is indicated in a document (Mikula-Pietrasik J Kuczmarska A, Kucin'ska M. Muriaset M. al. Resveratrol and its synthetic derivatives exert opposite effects on mesothelial cell-dependent angiogenesis via modulating secretion of VEGF and IL-8/CXCL8. Angiogenesis 2012; 15: 361-376, and above-described Keglowich 1 et al.).

MCP-1 is reported as a neovascularization chemokine, and the angiogenesis ability is also indicated in another one document (6. Niu J, Azfer A, Zhelyabovska O, Fatma S, and Kolattukudy PE. Monocyte Chemotactic Protein (MCP)-1 Promotes Angiogenesis via a Novel Transcription Factor, MCP-1-induced Protein (MCPIP). J Biol Chem2008 May 23;283(21): 14542-51.doi: 10.1074/jbc.M802139200). It is indicated in the document that MCP-1 mediates the angiogenesis ability of VEGF (3.Hong KH, Ryu J, Han KH. Monocyte Chemoattractant protein-1-induced Angiogenesis Is Mediated by Vascular Endothelial Growth factor-A. Blood 2005; 105:1405-1407 DOI:10,1182/blood-2004-08-3178). It is necessary for the formation of blood vessel to require macrophages migration to the transplanted sites, therefore MCP-1 is an essential factor for it.

Accordingly, it was shown that all of the four kinds of cytokines (MCP-1, IL-8, GRO, VEGF) secreted from the cultured chondrocyte into the conditioned medium to give examples of the angiogenesis ability.

## INDUSTRIAL APPLICABILITY

This invention is applicable in the field of medical device and the like.

## Claims

1. A method for producing a mature chondrocyte, comprising

    a culturing step of cartilages using a first culture medium to obtain a mature chondrocyte, wherein
    the first culture medium is a medium containing hydrocortisone and FGF-2.

2. The method for producing a mature chondrocyte according to claim 1, wherein
   the harvesting cartilage is an auricular cartilage.

3. The method for producing a mature chondrocyte according to claim 1, wherein
   the culturing step includes a step of culturing the cells under an environment of 2% or more and 15% or less of carbonic acid gas and 1% or more and 10% or less of oxygen gas against air.

4. A method for producing a composition containing mature chondrocyte, comprising:

    a culturing step of culturing cells from cartilage using a first culture medium to obtain a mature chondrocyte; and
    a composition containing mature chondrocyte obtaining step to get the mature chondrocyte and factors from it, wherein
    the first culture medium is a medium containing hydrocortisone and FGF-2.

5. The method for producing a composition containing mature chondrocyte according to claim 4, wherein
   the cartilage culturing step includes a step of culturing cells from cartilage under an environment of 2% or more and 15% or less of carbonic acid gas and 1% or more and 10% or less of oxygen gas against air.

6. The method for producing a composition containing mature chondrocyte according to claim 4, wherein
   the conditioned medium contains interleukin 8 (IL-8), a growth related gene (GRO), a monocyte chemotactic protein-1 (MCP-1), and VEGF.

7. The method for producing a composition containing mature chondrocyte according to claim 4, wherein
   the composition containing mature chondrocyte is used for transplantations, resulting in complete cartilage regeneration with perichondrium.

8. The method for producing a composition containing mature chondrocyte according to claim 4, wherein
   the composition containing mature chondrocyte is used for a pinnaplasty, an external ear canal construction, a rhinoplasty, a mandibular construction, a hard tissue depression construction operation of cheekbones, a hard tissue depression construction operation of skull, a hard tissue depression construction operation of trachea, funnel chest, or the like.

9. A method for producing a revascularization promoter, wherein

    the revascularization promoter contains a composition containing mature chondrocyte and cells that contains a vascular inducer and a VEGFR2 positive cell, and
    the method includes a step of producing the composition containing mature chondrocyte by the method for producing a composition containing mature chondrocyte according to claim 4.

Fig. 1

Fig. 2

Fig. 3

Fig. 4

A                       B

C                       D

Fig. 5

P1

P2

P3

P4

Fig. 6

Fig. 7

Fig. 8

Fig. 9

Fig. 10

## INTERNATIONAL SEARCH REPORT

International application No.

**PCT/JP2021/045015**

### A. CLASSIFICATION OF SUBJECT MATTER

***C12N 5/077***(2010.01)i
FI: C12N5/077

According to International Patent Classification (IPC) or to both national classification and IPC

### B. FIELDS SEARCHED

Minimum documentation searched (classification system followed by classification symbols)

C12N5/077

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2016/076102 A1 (REGENESIS SCIENCE CO LTD) 19 May 2016 (2016-05-19) claims, examples | 1-6 |
| A | | 7-9 |
| X | WO 2002/012451 A1 (YANAGA, Hiroko) 14 February 2002 (2002-02-14) examples | 1-6 |
| A | | 7-9 |
| X | JP 2006-314759 A (YANAGA, Hiroko) 24 November 2006 (2006-11-24) paragraph [0024] | 1-6 |
| A | | 7-9 |
| X | JP 2005-000143 A (YANAGA, Hiroko) 06 January 2005 (2005-01-06) paragraph [0020], examples | 1-6 |
| A | | 7-9 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 January 2022** | **15 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2021/045015** |

C.    DOCUMENTS CONSIDERED TO BE RELEVANT

| Category* | Citation of document, with indication, where appropriate, of the relevant passages | Relevant to claim No. |
|---|---|---|
| X | JP 2000-515023 A (GENZYME CORPORATION) 14 November 2000 (2000-11-14) claims | 1-6 |
| A | | 7-9 |
| X | WO 2005/105992 A1 (NEW YORK EYE & EAR INFIRMARY) 10 November 2005 (2005-11-10) claims, examples | 1-6 |
| A | | 7-9 |
| X | WO 1998/059035 A2 (THE GOVERNMENT OF THE UNITED STATES OF AMERICA, AS REPRESENTED BY THE SECRETARY. DEPARTMENT OF HEALTH AND HUMAN SERVICES NATIONAL INSTITUTES OF HEALTH. OFFICE OF TECHNOLOGY TRANSFER) 30 December 1998 (1998-12-30) claims, examples | 1-6 |
| A | | 7-9 |
| X | CN 104630138 A (SHAANXI RUISHENG BIOTECH CO LTD) 20 May 2015 (2015-05-20) claims, examples | 1-6 |
| A | | 7-9 |

Form PCT/ISA/210 (second sheet) (January 2015)

INTERNATIONAL SEARCH REPORT
Information on patent family members

International application No.

**PCT/JP2021/045015**

| Patent document cited in search report | | | Publication date (day/month/year) | Patent family member(s) | | | Publication date (day/month/year) |
|---|---|---|---|---|---|---|---|
| WO | 2016/076102 | A1 | 19 May 2016 | US | 2017/0306294 | A1 | |
| | | | | claims, examples | | | |
| | | | | EP | 3219792 | A1 | |
| | | | | CN | 106459912 | A | |
| WO | 2002/012451 | A1 | 14 February 2002 | US | 2003/0180943 | A1 | |
| | | | | examples | | | |
| | | | | EP | 1331264 | A1 | |
| JP | 2006-314759 | A | 24 November 2006 | US | 2006/0228423 | A1 | |
| | | | | paragraph [0037] | | | |
| | | | | KR | 10-2006-0107897 | A | |
| JP | 2005-000143 | A | 06 January 2005 | US | 2006/0088506 | A1 | |
| | | | | paragraph [0040], examples | | | |
| | | | | EP | 1619245 | A1 | |
| | | | | KR | 10-2005-0113283 | A | |
| | | | | CN | 1774502 | A | |
| JP | 2000-515023 | A | 14 November 2000 | US | 6150163 | A | |
| | | | | claims | | | |
| | | | | WO | 1998/004681 | A2 | |
| | | | | EP | 0920490 | A2 | |
| WO | 2005/105992 | A1 | 10 November 2005 | US | 2007/0178074 | A1 | |
| WO | 1998/059035 | A2 | 30 December 1998 | US | 2001/0039050 | A1 | |
| CN | 104630138 | A | 20 May 2015 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013018756 A
- JP 5139294 B
- JP 5526320 B

### Non-patent literature cited in the description

- **VACANTI CA ; LANGER R ; SCHLOO B ; VACANTI JP.** Synthetic polymers seeded with chondrocytes provide a template for new cartilage formation. *Plast Reconstr Surg.,* 1991, vol. 88, 753-759
- **CAO Y ; VACANTI JP ; PAIGE KT ; UPTON J ; VACANTI CA.** Transplantation of Chondrocytes Utilizing a Polymer-Cell Construct to Produce Tissue-Engineered Cartilage in the Shape of a Human Ear. *Plast Reconstr Surg.,* 1997, vol. 100, 297-302
- **SANTAVIRTA S ; KONTTINEN YT ; SAITO T et al.** Immune response to polyglycolic acid implants. *J Bone Joint Surg Br.,* 1990, vol. 72, 597-600
- **NEIDEL J ; ZEIDLER U.** Independent effects of interleukin 1 on proteoglycan synthesis and proteoglycan breakdown of bovine articular cartilage in vitro. *Agents Actions.,* 1993, vol. 39, 82-90
- **VACANTI CA.** The history of tissue engineering. *J Cell Mol Med,* 2006, vol. 10, 569-576
- **YANAGA H. ; IMAI K. ; KOGA M. ; YANAGA K.** Cell-engineered human elastic chondrocytes regenerate natural scaffold in vitro and neocartilage with neo-perichondrium in the human body post-transplantation. *Tissue Eng. Part A.,* 2012, vol. 18, 2020-2029
- **YANAGA H. ; IMAI K. ; FUJIMOTO T. ; YANAGA K.** Generating ears from cultured autologous auricular chondrocytes by using two-stage implantation in treatment of microtia. *Plast Reconstr Surg.,* 2009, vol. 124, 817-825
- **MIKULA-PIETRASIK J ; KUCZMARSKA A ; KUCIN'SKA M. ; MURIASET M.** Resveratrol and its synthetic derivatives exert opposite effects on mesothelial cell-dependent angiogenesis via modulating secretion of VEGF and IL-8/CXCL8. *Angiogenesis,* 2012, vol. 15, 361-376
- **NIU J ; AZFER A ; ZHELYABOVSKA O ; FATMA S ; KOLATTUKUDY PE.** Monocyte Chemotactic Protein (MCP)-1 Promotes Angiogenesis via a Novel Transcription Factor, MCP-1-induced Protein (MCPIP). *J Biol Chem,* 23 May 2008, vol. 283 (21), 14542-51
- **HONG KH ; RYU J ; HAN KH.** Monocyte Chemoattractant protein-1-induced Angiogenesis Is Mediated by Vascular Endothelial Growth factor-A. *Blood,* 2005, vol. 105, 1405-1407